# EUROPEAN PATENT APPLICATION

(11) **EP 4 603 086 A1**
(43) Date of publication of application: **20.08.2025**
(21) Application number: 22961699.0
(22) Date of filing: 12.10.2022
(51) Int. Cl.: A61K 31/122, A61K 45/06, A61P 29/00, A61P 37/08, A61P 17/00, A61P 1/00, A61P 19/02, A61P 25/00

(54) **PHARMACEUTICAL COMPOSITION FOR TREATING MAST CELL-MEDIATED INFLAMMATORY DISEASES**

(71) Applicant: Chu, Lei, Chengdu, Sichuan 610000 (CN)
(72) Inventor: LERNER, Ethan, Chengdu, Sichuan 610000 (CN); REDDY, Vemuri B, Chengdu, Sichuan 610000 (CN); RAN, Yuping, Chengdu, Sichuan 610000 (CN); CHU, Lei, Chengdu, Sichuan 610000 (CN)
(74) Representative: Plavsa, Olga
(86) International application number: PCT/CN2022/124774
(87) International publication number: WO 2024/077506

(57) **Abstract**

The present disclosure relates to use of Atovaquone for treating and preventing acute and chronic inflammatory diseases in a mammal. The acute and chronic inflammatory diseases comprise mast cell-mediated inflammatory diseases.

## Description

### Technical Field

The present disclosure relates to the field of medical technologies, and in particular, to the medical field of inflammatory diseases.

### Background

Inflammatory reactions are tissue cell reactions that occur in the body in response to pathogenic microbial infections, trauma, allergic reactions, etc. The inflammatory reactions are beneficial physiologically and pathophysiologically, which are necessary for wound healing as well as resolution and recovery of viral or bacterial infection. However, excessive inflammatory reactions are harmful, because they involve allergic reactions or quasi-allergic reactions, and allergic or idiopathic inflammation. A mast cell is the core for initiating and maintaining inflammation.

The mast cell, as one of key effector cells during the inflammatory reaction, is an important link between a nervous system and an immune system, and participates in multiple pathological processes such as postoperative pain and skin inflammation. Relative to other natural immune cells, the mast cell has significant space advantages, which is one of the cells first responding to sensory nerve fiber activation at a location closer to peripheral nerve endings. The mast cell activated by a neuropeptide releases multiple "proinflammatory cytokines" and "chemokines", and participates in the recruitment of multiple natural immune cells, thereby further promoting inflammatory cascade reactions and peripheral sensory afferent sensitization.

The peripheral nervous system of the body has a function of receiving external environment change stimuli and conducting information into a central nervous system to generate various sensations. Various receptors distributed on the peripheral nervous system play important roles. G protein coupled receptors are a class of the many receptors that play important roles in sensations of smell, taste, pain, itching and the like. The function of Mas-related G protein coupled receptors (Mrgprs), as a recently discovered G protein coupled receptor family that is mainly distributed on the peripheral nervous system, has attracted increasing attentions from researchers in related fields. Mrgprs are a class of Mas gene related G protein coupled receptors. By analyzing a hydrophobicity profile of a Mas amino acid sequence and utilizing an algorithm that correctly predicts multiple protein transmembrane fragments, Young et al. have found that the Mrgpr has seven hydrophobic transmembrane domains, indicating that this protein belongs to the G protein coupled receptors (GPCRs).

MrgprX2 is initially found to have a high expression level in a human dorsal root ganglion (DRG) small-diameter nociceptive sensory neuron, predicting that MrgprX2 plays a key role in nociception; MrgprX2 is found to have a high expression level in mast cells; the latest researches show that MrgprX2 is also expressed in human eosinophils and basophils and mediates the degranulation of MrgprX2. The mast cell, as one of key effector cells in an immune reaction process, is an important link connecting a nervous system with an immune system. Since the mast cells can be extremely close to nerve endings, they have significant spatial advantages relative to other innate immune cells and can respond to sensory nerve activation first. When the mast cells are activated, they release a wide range of proinflammatory cytokines and chemokines to act on specific receptors on sensory nerve endings, causing the nerve endings to release neuropeptides such as substance P (SP) and a vasoactive intestinal peptide (VIP). These neuropeptides activate receptors on the mast cells to induce the further degranulation of the mast cells to release a large amount of proinflammatory cytokines and chemokines, causing neurogenic inflammation. Except for local interaction between the mast cells and sensory nerves, a pain signal may also be amplified through a nerve ending Ca²⁺ -mediated axon potential to cause the excitement of peripheral fibers so that the generated neural signal can travel along axons to reach the central nervous system; peptide substances such as neurogenic SP and VIP activate the mast cells through the MrgprX2 receptor, which indicates that MrgprX2 contributes to the development of neurogenic inflammation and pain.

Recent studies have confirmed that in a postoperative pain model and a CFA induced inflammatory pain model, the inflammation and hyperalgesia reaction in MrgprB2-/-mice are significantly alleviated compared to those in normal mice. The recruitment of innate immune cells in MrgprB2-/-mice at an injury site is significantly reduced, and a hypothesis that mast cells interact with nerve cells has been proposed: SP released after tissue injury activates the mast cells through MrgprB2 to cause the release of cytokines and chemokines and the recruitment of immune cells, thereby participating in inflammation and pain. With the deep research of MrgprB2/MrgprX2, it has been found that the function of MrgprB2/MrgprX2 is becoming increasingly important, this receptor not only widely participates in body's reactions such as non-IgE mediated hypersensitivity reactions, neurogenic inflammation, pain and convulsion, but also can promote innate immune reactions of skins and intestines to harmful stimuli or pathogen invasion, and therefore this receptor is considered as a powerful target for seeking anti-inflammatory and analgesic drugs for development.

### Summary

The present disclosure provides a drug for treating or preventing acute and chronic inflammatory diseases in a mammal, wherein the acute and chronic inflammatory diseases comprise mast cell-mediated inflammatory diseases. The drug is Atovaquone.

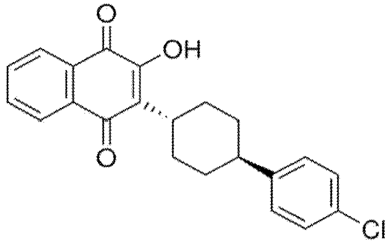

Atovaquone, i.e., hydroxy 1,4-naphthaline, is a naphthoquinone compound, which is a homologue of coenzyme Q and has the activity against several protozoa. The acting site of Atovaquone on plasmodium is a cytochrome B-cell lymphoma (BCL) binding site (binding site III). The drug reversely binds to a 11500 Da molecular group on a polypeptide. A dihydroorotate dehydrogenase is an important enzyme in pyridine biosynthesis, which is connected with mitochondrions through coenzyme Q for electron transfer, so as to prevent the synthesis of pyridine by inhibiting the electron transfer. Some metabolic enzymes participate in the electron transfer of mitochondrions through coenzyme Q, the inhibition of Atovaquone on electron transfer is indeed the inhibition of the activity of these enzymes, and therefore Atovaquone has a broad-spectrum anti-protozoon activity.

Although the broad-spectrum anti-protozoon activity of Atovaquone has been widely studied, there are few of researches on other pharmacological activities of Atovaquone. The present disclosure provides new uses of Atovaquone for treating and preventing various diseases involving excessive inflammatory reactions by inhibiting the degranulation of mast cells. These uses involve effective treatment and prevention of various inflammatory diseases involving mast cells. Such the inflammatory diseases include, but are not limited to, allergies and atopic disorders (allergic asthma, eczema, and rhinitis), mast cell activation syndrome (MCAS), physical and chemical urticaria, idiopathic urticaria, Crohn's disease, inflammatory bowel diseases, dermatitis and contact dermatitis, arthritis and rheumatoid arthritis, skin, tissue, or systemic reactions to stinging or other allergic or quasi-allergic stimuli, canine mastocytosis, allergies and inflammation in cattle and pigs, etc. The inflammatory diseases further involve allergic dermatitis or other allergic diseases such as skin eczema, dermatitis, atopic dermatitis, contact dermatitis, neurodermatitis and urticaria in adults, children or infants.

At present, the use of a corticosteroid therapy to treat and/or prevent excessive inflammatory reactions has obvious side effects and use dependence. However, report results from a large number of users in a population show that Atovaquone has low side effect characteristics and no significant drug dependence phenomenon after long-term use.

Therefore, the present disclosure has an important value in treatment and prevention of various inflammatory diseases.

One aspect of the present disclosure provides a pharmaceutical composition, comprising an active ingredient Atovaquone.

The pharmaceutical composition treats and prevents various diseases involving excessive inflammatory reactions by inhibiting the degranulation of mast cells. Such the inflammatory diseases include, but are not limited to, allergies and atopic disorders, mast cell activation syndrome (MCAS), physical and chemical urticaria, idiopathic urticaria, Crohn's disease, inflammatory bowel diseases, dermatitis and contact dermatitis, arthritis and rheumatoid arthritis, skin, tissue or systemic reactions to stinging or other allergic or quasi-allergic stimuli, canine mastocytosis, allergies and inflammation in cattle and pigs, etc. The inflammatory diseases further include allergic dermatitis or other allergic diseases such as skin eczema, dermatitis, atopic dermatitis, contact dermatitis, neurodermatitis and urticaria in adults, children or infants.

Some embodiments of the present disclosure relate to a pharmaceutical composition. The pharmaceutical composition comprises an active pharmaceutical ingredient Atovaquone and a pharmaceutically acceptable carrier or diluent.

In some embodiments, the pharmaceutical composition is an emulsifiable paste, an emulsion, a cream, a solution, a patch, a gel, an aerosol, a tablet, a granule, an injection, etc.

In some embodiments, the pharmaceutical composition is formulated for administration by inhalation, by an evaporator, by a sprayer, or by an aerosolizer. In some embodiments, the pharmaceutical composition is formulated for oral administration, oral cavity administration or sublingual administration. In some embodiments, the pharmaceutical composition is formulated for intravenous, intramuscular or subcutaneous administration. In some embodiments, the pharmaceutical composition is formulated for intrathecal or intracerebroventricular administration. In some embodiments, the pharmaceutical composition is formulated for topical administration.

In some embodiments, the active pharmaceutical ingredient is present in the pharmaceutical composition at a concentration of 0.0001-100 mg/ml, preferably 0.0001-50 mg/ml.

In another aspect, the present disclosure provides a method for treating immune system diseases. The method comprises administrating an effective amount of the pharmaceutical composition of the present disclosure.

In some embodiments, the immune system diseases are allergies or atopic disorders. In some embodiments, the immune system diseases are mast cell activation syndrome (MCAS), physical and chemical urticaria, idiopathic urticaria, Crohn's disease, inflammatory bowel diseases, dermatitis and contact dermatitis, arthritis and rheumatoid arthritis, skin, tissue or systemic reactions to stinging or other allergic or quasi-allergic stimuli, canine mastocytosis, allergies and inflammation in cattle and pigs, etc. The inflammatory diseases further involve allergic dermatitis or other allergic diseases such as skin eczema, dermatitis, atopic dermatitis, contact dermatitis, neurodermatitis and urticaria in adults, children or infants. Allergic inflammatory diseases in respiratory and digestive systems include, but are not limited to, asthma, rhinitis, allergic pneumonia, allergic enteritis, etc.

In some embodiments, the immune diseases are diseases involving the dysregulation of a CB1 or CB2 receptor, or the cAMP dysregulation of mast cells. In some embodiments, the immune diseases are diseases involving the excessive activation of the CB1 or CB2 receptor, or the cAMP inhibition of mast cells.

In some embodiments, the pharmaceutical composition is administrated by inhalation. In some embodiments, the pharmaceutical composition is orally administrated. In some embodiments, the pharmaceutical composition is administrated by oral cavity administration. In some embodiments, the pharmaceutical composition is delivered by subligngual administration. In some embodiments, the pharmaceutical composition is administrated by injection. In some embodiments, the pharmaceutical composition is administrated by topical administration, preferably by topical skin administration.

In some embodiments, Atovaquone is administrated in an amount of less than 1.5 mg per dose. In some embodiments, Atovaquone is administrated in an amount of less than 1000 ng per dose. In some embodiments, Atovaquone is administrated in an amount of less than 500 ng per dose. In some embodiments, Atovaquone is administrated in an amount of less than 100 ng per dose. In some embodiments, Atovaquone is administrated in an amount of less than 10 ng per dose.

In some embodiments, if necessary, the pharmaceutical composition is administrated. In some embodiments, the pharmaceutical composition is administrated once a day. In some embodiments, the pharmaceutical composition is administrated 2-4 times a day. In some embodiments, the pharmaceutical composition is administrated 2-4 times a week. In some embodiments, the pharmaceutical composition is administrated once a week. In some embodiments, the pharmaceutical composition is administrated once every two weeks.

Next, these and other aspects of the present disclosure will be described in detail

### Brief Description of the Drawings

FIG. 1 shows the inhibition of a degranulation percentage of mast cells by a drug.
FIG. 2 is a dynamic observation graph of calcium ion changes when substance P acts on different receptors.
FIG. 3 shows a calcium ion flow curve at different drug concentrations.

### Detailed Description of the Embodiments

### Active pharmaceutical ingredient

In a first aspect, the present disclosure provides a pharmaceutical composition. The main active ingredient of the pharmaceutical composition is Atovaquone.

### Pharmaceutical composition

In another aspect, the present disclosure provides a pharmaceutical composition. The pharmaceutical composition comprises an active ingredient Atovaquone and a pharmaceutically acceptable carrier or diluent. The pharmaceutical composition further comprises another active ingredient, i.e., a second active substance, which is different from Atovaquone. The second active substance is selected from the group consisting of antihistamine drugs, corticosteroid hormone drugs, mast cell stabilizers (such as sodium cromoglycate), leukotriene receptor antagonists (such as Montelukast), antimicrobial infection drugs (Fungal, bacterial, viral, chlamydial, mycoplasma infections and the like, such as Itraconazole), drugs containing a licorice extract active ingredient (such as compound glycyrrhizin, and diammonium glycyrrhizinate), calcineurin inhibitors (such as Tacrolimus and Pimecrolimus), immunosuppressants (such as cyclosporine, azathioprine, methotrexate), monoclonal antibodies (such as Dupilumab), rheumatoid immune drugs for treating allergic inflammation, etc.

### The content of active pharmaceutical ingredient

In a typical embodiment, the active ingredient is present in the pharmaceutical composition at a concentration of at least 0.001 µg/ml, at least 0.01 µg/ml, at least 0.5 µg/ml or at least 1 µg/ml. In some embodiments, the active ingredient is present in the pharmaceutical composition at a concentration of at least 1 µg/ml, 2 µg/ml, 3 µg/ml, 4 µg/ml, 5 µg/ml, 10 µg/ml, 15 µg/ml, 20 µg/ml or 25 µg/ml. In some embodiments, the active ingredient is present in the pharmaceutical composition at a concentration of at least 30 µg/ml, 35 µg/ml, 40 µg/ml, 45 µg/ml or 50 µg/ml.

### General drug formulation

The pharmaceutical composition is in any form suitable for human or veterinary use, including an emulsifiable paste, an emulsion, a cream, a solution, a patch, a gel, an aerosol, a tablet, a granule, an injection, etc.

The pharmaceutical composition is formulated for administration through any administration routes suitable for human or veterinary use, including intestinal and gastrointestinal administration routes.

In various embodiments, the pharmaceutical composition is formulated for administration through inhalation; the pharmaceutical composition is formulated for oral administration, oral cavity administration or sublingual administration. The pharmaceutical composition is formulated for intravenous, intramuscular or subcutaneous administration; the pharmaceutical composition is formulated for topical administration, for example topical skin administration, and topical mucosa administration.

A mucosal preparation is administrated in a form of dry powder preparation, for example, comprising a bioactivator which is a dry form with an appropriate particle size or in an appropriate particle size range, generally a freeze-drying form, for intranasal delivery. The minimum particle size suitable for deposition in a nasal or pulmonary passage is typically about 0.5 µm of mass median equivalent aerodynamic diameter (MMEAD), typically about 1 µm of MMEAD, more typically about 2 µm of MMEAD. The maximum particle size suitable for deposition in a nasal cavity is typically about 10 µm of MMEAD, typically about 8 µm of MMEAD, more typically about 4 µm of MMEAD. Nasal inhalable powders within these size ranges are produced by various conventional technologies such as spray grinding, spray drying, solvent precipitation and supercritical fluid condensation. These appropriate MMEAD dry powders are administrated into patients through a conventional dry powder inhaler (DPI). The DPI relies on patient's breath to disperse powders into an atomized amount after pulmonary or nasal inhalation. Alternatively, the dry powders are administrated by an air assisted device. The device disperses the powders into the atomized amount by using an external power supply such as a piston pump.

### Pharmaceutical composition suitable for oral/oral cavity/sublignual administration

A preparation suitable for oral/oral cavity/sublignual administration is a capsule, a cachet, a pill, a tablet, a lozenge (which uses a flavored base, which is typically sucrose and arabic gum or tragacanth gum), a powder, a granule, or serves as a solution or suspension in an aqueous or non-aqueous liquid, or serves as an oil-in-water or water-in-oil liquid emulsion, or serves as a melting agent or syrup, or serves as a troche (which uses an inert base, such as gelatin and glycerinum, or sucrose and arabic gum), and/or serves as a mouthwash, etc., each of which contains a predetermined amount of subject polypeptide therapeutic agent as an active ingredient. Except for an active compound, the suspension also contains a suspending agent, such as ethoxylated isostearyl alcohol, polyoxyethylene sorbitol and sorbitan ester, microcrystalline cellulose, aluminium metahydroxide, bentonite, agar and tragacanth gum, and a mixture thereof.

In a solid dosage form (a capsule, a tablet, a pill, a dragee, a powder, a granule, etc.) for oral, oral cavity or sublingual administration, one or more therapeutic agents are mixed with one or more pharmaceutically acceptable carriers, for example sodium citrate or dicalcium phosphate, and/or any one selected from the group consisting of: (1) a filler or an incremental agent, such as starch, lactose, sucrose, glucose, mannitol, and/or silicic acid; (2) an adhesive, such as carboxymethyl cellulose, alginate, gelatin, polyvinylpyrrolidone, sucrose, and/or arabic gum; (3) a humectant, such as glycerinum; (4) a disintegrating agent, such as agar, calcium carbonate, potato starch or cassava starch, alginic acid, some silicates and sodium carbonate; (5) a solution retarder, such as paraffin; (6) an absorption enhancer, such as a quaternary ammonium compound; (7) a wetting agent, such as whale wax alcohol and glycerol monostearate; (8) an absorbent, such as kaolin and bentonite; (9) a lubricating agent, such as talc, calcium stearate, magnesium stearate, solid polyethylene glycol, sodium dodecyl sulfate and a mixture thereof; and (10) a coloring agent. In the case of capsules, tablets and pills, the pharmaceutical composition also comprises a buffer. Similar types of solid compositions may also be used as fillers in soft and hard-filled gelatin capsules, which use an excipient such as lactose, and high molecular weight polyethylene glycol, etc. A liquid dosage form for oral administration includes a pharmaceutically acceptable emulsion, a microemulsion, a solution, a suspension, a syrup and a melting agent. Except for the active ingredient, the liquid dosage form also contains an inert diluent commonly used in the art, such as water or other solvents, a solubilizer and an emulsifier such as ethanol, isopropanol, ethyl carbonate, ethyl acetate, benzyl alcohol, benzyl benzoate, propylene glycol, 1,3-butanediol, oil (especially cottonseed oil, peanut oil, corn oil, germ oil, olive oil, castor oil, and sesame oil), glycerol, tetrahydrofuran, polyethylene glycol, and a fatty acid ester of dehydrated sorbitol, as well as a mixture thereof. Except for the inert diluent, the oral composition also comprises an auxiliary agent, such as a wetting agent, an emulsifier and a suspending agent, a sweetening agent, a flavoring agent, a coloring agent, an aromatic and a preservative.

### Pharmaceutical composition suitable for injection

For intravenous, intramuscular or subcutaneous injection, or injection at the affected area, the active ingredient will be in a form of gastrointestinally acceptable aqueous solution, which is pyrogen-free, and has suitable pH, isotonicity and stability. Those skilled in the art can use an isotonic medium (such as a sodium chloride injection, a Ringer's injection and a lactic acid Ringer's injection) to prepare an appropriate solution. As needed, a preservative, a stabilizing agent, a buffer, an antioxidant and/or other additives are included therein.

In various embodiments, a unit dosage form is a vial, an ampoule, a bottle, or a pre-filled syringe. In some embodiments, the unit dosage form contains 0.01 µg, 0.1 µg, 0.5 µg, 1 µg, 2.5 µg, 5 µg, 10 µg, 12.5 µg, 25 µg, 50 µg, 75 µg or 100 µg of Atovaquone composition. In some embodiments, the unit dosage form contains 125 µg, 150 µg, 175 µg or 200 µg of Atovaquone composition. In some embodiments, the unit dosage form contains 250 µg of Atovaquone composition.

In some embodiments, the pharmaceutical composition in the unit dosage form is a solid form suitable for dissolution, for example a lyophilizate.

The pharmaceutical composition and preparation suitable for topical administration may comprise a transdermal patch, an ointment, a lotion, an emulsified paste, a gel, a drop, a suppository, a spray, a liquid and a powder. The conventional drug carrier, a water-based, powdery or oily base, a thickening agent and the like may be necessary or expected.

### General dose range

An in-vivo and/or in-vitro assay is optionally used to help the determination of the used optimal dose range. The precise dose used in the preparation also depends on an administration route and severity of a disease, and should be determined according to physician's judgment and individual situations. An effective dose beyond a dose-reaction curve of a system is tested according to an in-vitro or animal model.

### Use method: a method for treating a mast cell-mediated inflammatory disease

In another aspect, provided is a method for treating an individual suffering from an immune system disease. In a typical embodiment, the immune system disease is a mast cell related inflammatory disease, the treated disease includes, but is not limited to, mast cell activation syndrome (MCAS), physical and chemical urticaria, idiopathic urticaria, Crohn' s disease, inflammatory bowel diseases, dermatitis and contact dermatitis, arthritis and rheumatoid arthritis, skin, tissue, or systemic reactions to stinging or other allergic or quasi-allergic stimuli, canine mastocytosis, allergies and inflammation in cattle and pigs, etc. The immune system disease further involves skin eczema, dermatitis, atopic dermatitis, contact dermatitis, neurodermatitis, urticaria and the like in adults, children or infants. Allergic inflammatory diseases of a respiratory system and a digestive system include, but are not limited to, asthma, rhinitis, allergic pneumonia, allergic enteritis, etc.

The terms "treatment" and the like used herein typically refer to obtaining required pharmacological and/or physiological effects. As to completely or partially prevent diseases, disorders or symptoms thereof, the effect can be preventive, and/or as to partially or completely cure the diseases or conditions and/or attributable to adverse reactions (such as symptoms) of the diseases or conditions, the effect can be therapeutic. The term "treatment" used herein includes any treatment for diseases or conditions of mammals, especially human, and includes: (a) preventing the occurrence of the diseases or conditions in an individual who is susceptible to the diseases or conditions but has not yet been diagnosed with the diseases or conditions; (b) inhibiting the diseases or conditions (for example, preventing its progression); or (c) relieving the diseases or conditions (for example, causing the subsidence of the diseases or conditions, and providing an improvement in one or more symptoms). The improvement of any conditions is evaluated according to standard methods and technologies known in the art. An individual group who is treated through the method includes individuals suffering from undesirable conditions or diseases, and individuals at the risk of suffering from the conditions or diseases.

The term "therapeutically effective dose" or "effective amount" refers to a dose or amount administrated to produce the desired effect. The exact dose or amount depends on a therapeutic purpose, and is determined by those skilled in the art based on a known technology.

In some embodiments, the pharmaceutical composition is administrated once a day, 2-4 times a day, 2-4 times a week, once a week or once every two weeks.

Depending on the condition to be treated, the composition can be administrated alone, or in combination with other treatments simultaneously or successively.

The present disclosure provides a pharmaceutical composition comprising Atovaquone. It has been proven that this composition has a significant anti-inflammatory effect, and therefore has therapeutic effects on immune conditions involving excessive inflammatory reactions, for example the release of abnormally increased histamine from mast cells.

### Examples

The provision of the following examples is for illustration but not limitation.

### Example 1: Study on inhibition of Atovaquone on mast cell degranulation

An experimental method using a β-hexosaminidase assay comprises the following steps:
1. a PNAG/HEPES buffer/0.1%Triton-100/glycine solution was prepared at a normal temperature;
2. LAD2cell was taken out from an incubator at 37°C and cell count was read, and then an appropriate amount of cell suspension was taken according to experimental cell requirements (estimated based on 5000-10000/well);
3. the LAD2 cell suspension was centrifuged for 5 min on a centrifuge at 4°C at 3000 rps, and then supernatant was discarded;
4. cells were evenly mixed in the solution after HEPES buffer was added;
5. the cell suspension was transferred to a 96-well plate, with 80 µl per well;
6. a to-be-tested drug Atovaquone was added to a corresponding position according to experiment design, the positions of a positive control SP (neuropeptide substance P), a negative control QWF (MRGPRX2 receptor antagonist) and a blank control were reserved, and each drug was administrated for three repetitions;
7. the cells were co-incubated with the drug for 30 min;
8. SP was added into all plates except the blank control, and then incubated for 30 min;
9. the above incubated product was centrifuged for 5 min at 3000 rps at a low temperature, and then 50 µ1 of supernatant was taken and added into a supernatant plate added with 50 µ1/well PNAG in advance for 90 min of co-incubation;
10. 150 µ1/well 0.1%Triton-100 was added into the solution remained after the supernatant was taken, and then the mixed solution described above was taken and added into a cell lysis plate added with 50 µ1/well PNAG in advance for 90 min of co-incubation;
11. a 50 µ1/well glycine solution was added respectively after the two plates were taken;

### Detection of m absorbance

12. the degranulation percentage of mast cells was calculated.

According to a formula: degranulation percentage=2×absorbance of supernatant plate/(absorbance of supernatant plate+4×absorbance of cell lysis plate)×100%

### Experimental results:

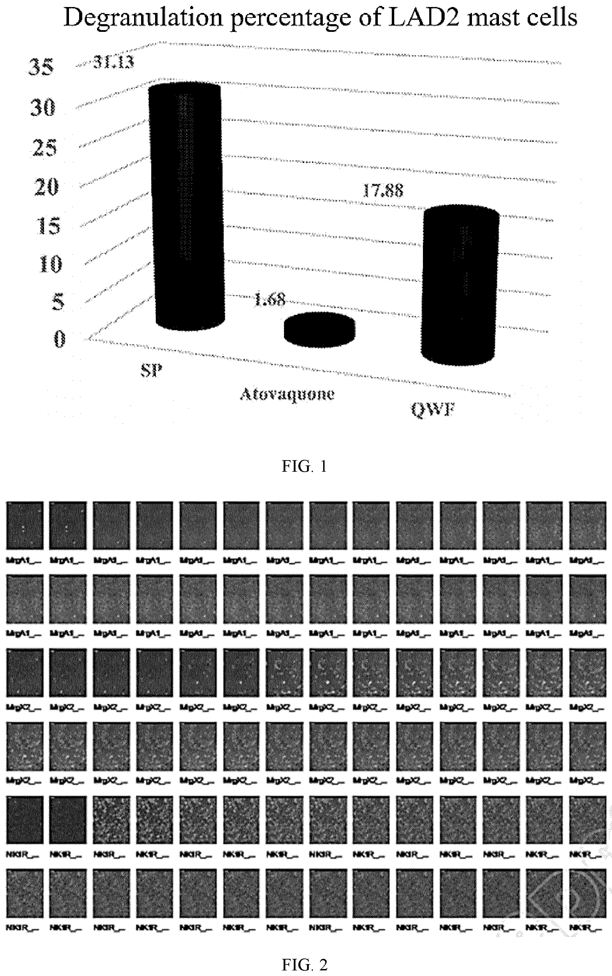

By the degranulation percentage graph of LAD2 mast cells in FIG. 1, it can be seen that in an SP stimulating experiment of LAD2 cells, the degranulation of LAD2 mast cells was obviously improved when Atovaquone and QWF were added, wherein Atovaquone was far superior to QWF. That is to say, Atovaquone is capable of significantly inhibiting the degranulation of mast cells.

### Example 2: intracellular calcium ion flow experiment

An incubation buffer was composed of: 0.5 µl of Fluo-3, 2 µl of Pluronic F-127 and 997.5 µl of CIB (calcium imaging buffer: 125 mM NaCl, 3 mM KCl, 2.5 mM CaCl₂, 0.6 mM MgCl₂, 10 mM HEPES, 20 mM glucose, 1.2 mM NaHCO₃ and 20 mM sucrose, and pH=7.4). Different concentrations (0, 1, 10, 100 nM and 1 µM) of Atovaquone were prepared in the incubation buffer, and an incubation buffer solution without Atovaquone was used as a negative control. LAD2 cells were washed twice with CIB, and then a desired incubation buffer solution was added. After incubation for 30 min, the cells were washed twice with CIB, and then inoculated into a 96-well plate for calcium imaging. For calcium imaging, the cells were magnified 200x and a photo was obtained per second under blue light. After a substance was injected, the cells were confirmed as being responsive if [Ca²⁺] rose by at least 50%.

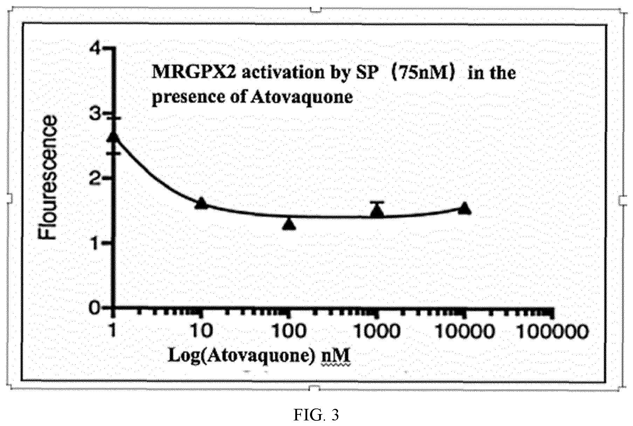

Experimental results are seen in FIG. 2 and FIG. 3.

FIG. 2 is a dynamic observation graph showing a calcium ion flow change when a substance P acted on different receptors, i.e., a schematic representation of the substance P acting on MrgA1, MrgX2 and NK1R (5 nM acted on NK1R, 50 nM acted on MrgX2 and 10 µM acted on MrgA1). This image set was photographed every 5 s from left to right for 120 s. In each group of graphs, a column represented cells with Atovaquone, the next column represented cells without Atovaquone. After Atovaquone was added, the effect on MrgA1 and NK1R mediated calcium ion flow was inferior to that on MrgX2.

FIG. 3 showed that Atovaquone at different concentrations leaded to reduction in calcium ion flow, and calcium ions began to show no obvious mobility when the concentration of Atovaquone was 10 nM/L and more.

It can be seen that Atovaquone significantly reduced the inflammatory activity of mast cells.

## Claims

1. A pharmaceutical composition for treating or preventing mast cell-mediated inflammatory diseases, wherein the pharmaceutical composition comprises an active ingredient Atovaquone.

2. The pharmaceutical composition according to claim 1, wherein the concentration of the active ingredient Atovaquone is 0.0001-100 mg/ml.

3. The pharmaceutical composition according to claim 1 or 2, wherein the pharmaceutical composition further comprises a second active ingredient.

4. The pharmaceutical composition according to any one of claims 1-3, wherein the pharmaceutical composition comprises a pharmaceutically acceptable carrier.

5. The pharmaceutical composition according to any one of claims 1-4, wherein the pharmaceutical composition is an emulsified paste, an emulsion, a cream, a solution, a patch, a gel, an aerosol, a tablet, a granule, or an injection.

6. Use of Atovaquone in a pharmaceutical composition for treating or preventing mast cell-mediated inflammatory diseases.

7. The use according to claim 6, wherein the inflammatory diseases comprise allergies and atopic disorders, mast cell activation syndrome (MCAS), physical and chemical urticaria, idiopathic urticaria, Crohn's disease, inflammatory bowel diseases, dermatitis and contact dermatitis, arthritis and rheumatoid arthritis, skin, tissue, or systemic reactions to stinging or other allergic or quasi-allergic stimuli, canine mastocytosis, and allergies and inflammations in cattle and pigs.

8. The use according to claim 7, wherein the inflammatory diseases further comprise skin eczema, dermatitis, atopic dermatitis, contact dermatitis, neurodermatitis and urticaria in adults, children or infants.

9. The use according to any one of claims 6-8, wherein the pharmaceutical composition is administrated systemically or topically.
